(19) Europäisches Patentamt
European Patent Office
Office européen des brevets

(11) **EP 1 971 292 B1**

(12) **EUROPEAN PATENT SPECIFICATION**

(45) Date of publication and mention
of the grant of the patent:
**11.10.2017 Bulletin 2017/41**

(21) Application number: **07709954.7**

(22) Date of filing: **04.01.2007**

(51) Int Cl.:
***A61F 2/16*** (2006.01)

(86) International application number:
**PCT/US2007/060112**

(87) International publication number:
**WO 2007/084800 (26.07.2007 Gazette 2007/30)**

(54) **ACCOMMODATING DIFFRACTIVE INTRAOCULAR LENS**

AKKOMMODATIONSFÄHIGE DIFFRAKTIVE INTRAOKULARLINSE

LENTILLE INTRAOCULAIRE DIFFRACTIVE A ACCOMMODATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.01.2006 US 331650**

(43) Date of publication of application:
**24.09.2008 Bulletin 2008/39**

(73) Proprietor: **Abbott Medical Optics Inc.
Santa Ana, CA 92705-4933 (US)**

(72) Inventors:
• **BRADY, Daniel, G.
San Juan Capistrano, CA 92675 (US)**
• **PIERS, Patricia, A.
NL-9717JA Groningen (NL)**

(74) Representative: **Hoffmann Eitle
Patent- und Rechtsanwälte PartmbB
Arabellastraße 30
81925 München (DE)**

(56) References cited:
**WO-A-2005/011531     US-A- 5 366 502**

**Description**

BACKGROUND OF THE INVENTION

FIELD OF THE INVENTION

The present invention is directed to accommodating intraocular lenses,

DESCRIPTION OF THE RELATED ART

A human eye can suffer diseases that impair a patient's vision. For instance, a cataract may increase the opacity of the lens, causing blindness. To restore the patient's vision, the diseased lens may be surgically removed and replaced with an artificial lens, known as an intraocular lens, or IOL.

The simplest IOLs have a single focal length, or, equivalently, a single power. Unlike the eye's natural lens, which can adjust its focal length within a particular range in a process known as accommodation, these single focal length IOLs cannot accommodate. As a result, objects at a particular position away from the eye appear in focus, while objects at an increasing distance away from that position appear increasingly blurred.

An improvement over the single focal length IOLs is an accommodating IOL, which can adjust its power within a particular range. As a result, the patient can clearly focus on objects in a range of distances away from the eye, rather than at a single distance. This ability to accommodate is of tremendous benefit for the patient, and more closely approximates the patient's natural vision than a single focal length IOL.

When the eye focuses on a relatively distant object, the lens power is at the low end of the accommodation range, which may be referred to as the "far" power. When the eye focuses on a relatively close object, the lens power is at the high end of the accommodation range, which may be referred to as the "near" power. The accommodation range itself is defined as the near power minus the far power. In general, an accommodation range of 4 diopters is considered sufficient for most patients.

The human eye contains a structure known as the capsular bag, which surrounds the natural lens. The capsular bag is transparent, and serves to hold the lens and impart a shape to the lens. In the natural eye, accommodation is initiated by the ciliary body and a series of zonular fibers, also known as zonules. The zonules are located in a relatively thick band around the equator of the lens, and impart a force from the ciliary body (collectively a zonule force or zonular force) to the capsular bag that can distort the natural lens and thereby change its power.

In a typical surgery in which the natural lens is removed from the eye, the lens material is typically broken up and vacuumed out of the eye, but the capsular bag is left intact. The remaining capsular bag is extremely useful for an accommodating intraocular lens, in that the eye's natural accommodation is initiated via the the ciliary body and zonules through the capsular bag. The capsular bag may be used to house an accommodating IOL, which in turn can distort and/or shift in some manner to affect the power and/or the axial location of the image.

The zonules may exert a force on the capsular bag up to about 10 grams of force, often between about 6 grams of force and about 9 grams of force, which is distributed typically generally uniformly around the equator of the capsular bag. The intraocular lens distorts and changes its power in response to this limited force, which is all that is available for accommodation. In general, it is desirable that the lens converts the zonule force to a power change in an efficient manner, so that the limited zonule force can subtend the entire accommodation range. In other words, a relatively small zonule force should initiate a relatively large change in power of the intraocular lens.

Early attempts at accommodating intraocular lenses used optics that were the essentially the size of the eye's natural lens. These optics are sometimes known as full size optics, and they occupy essentially the entire capsular bag. Although full size optics produce a change in power in response to the zonules force, the change is typically too small to cover the entire accommodation range. There is simply too much material in a full size lens; the full accommodation range requires a distorting force that is larger than the limited force produced by the zonules.

More recent attempts at accommodating intraocular lenses commonly use smaller and thinner optics (and typically refractive optics), which are more easily deformed by the limited zonules force. These optics, which are thinner and smaller than the capsular bag, will be referred to hereinafter as deformable optics.

These intraocular lenses may have discrete nominal power values ranging from 6 diopters to 33 diopters, with increments of 0.5 diopters. For each lens, the nominal condition is the relaxed condition, or, equivalently, the condition at which the lens is manufactured. An equivalent term is the lens bias. For an "accommodative biased" lens, the lens is manufactured so that in its relaxed state in the eye, it brings near objects into focus. For instance, if a 20 diopter lens is required for the patient, and objects are assumed to be at distances where 4 diopters of accommodation is sufficient to bring them into focus, then a suitable accommodative biased lens is manufactured with 24 diopters of power. Likewise, a "disaccommodative biased" lens is manufactured so that in its relaxed state in the eye, it brings distant objects into focus. For example, if a 20 diopter lens is required for the patient, then a suitable disaccommodative biased lens is manufactured with 20 diopters of power. For both of these numerical examples, the nominal power is 20 diopters and the accommodation range is 4 diopters. In general, a continuum of states between accommodative biased and disaccommodative biased is possible.

For each power value, there is typically an optimal geometry that balances a number of conditions, including optical performance at the retina, tolerances in manufacturing and alignment, cost, stability, and so forth. Although the specific radii and thickness may be chosen from a wide range of values, there are various trends that emerge, which are discussed below.

At low nominal powers, the preferred optic shape is meniscus, with a convex anterior surface and a concave posterior surface. This meniscus shape is well suited to the type of accommodation in which the optic is distorted, and the full accommodation range may generally be reached for suitable choices of the optic geometry.

At medium nominal powers, the preferred optic shape is close to piano-convex, with a convex anterior surface and a posterior surface that may be slightly convex, flat or slightly concave. This essentially piano-convex shape is less well suited to the type of accommodation in which the optic is distorted, and generally provides less than the full desired accommodation range for the limited zonule force.

At high nominal powers, the preferred optic shape is bi-convex. Like the plano-convex shape, the bi-convex lenses also generally provide less than the full desired accommodation range for the limited zonule force.

It should be noted that the generalizations made for the low/medium/high nominal power categories apply to similarly sized lenses. Compared with differently shaped lenses of a comparable size, a meniscus lens generally provides a larger range of accommodation for a given zonule force.

A drawback to this type of accommodating intraocular lens, in which the zonular force distorts the optic and thereby changes its power, is as follows. If the optic geometry is optimized for each nominal power (or for one or more particular subsets of the nominal power range), then the full range of accommodation cannot be reached for particular nominal powers. In particular, a meniscus lens that serves the low nominal powers can cover the full accommodation range, while similarly sized plano-convex or bi-convex lenses that serve the medium and high nominal powers cannot cover the full accommodation range.

Accordingly, there exists a need for a style of optic that can cover the full accommodation range, for the entire range of nominal powers.

WO 2005/011531 relates to an intraocular lens system including a primary intraocular lens configured to correct vision in a patient, and a supplemental intraocular lens configured to modify the correction provided by the primary intraocular lens. The suppplemental intraocular lens, which is substantially completely diffractive, is preferably ultrathin.

BRIEF SUMMARY OF THE INVENTION

The invention is defined by the features of the independent claims 1, 6, and 7. An embodiment of the present invention is a method of providing a range of nominal powers for a plurality of intraocular lenses, the method comprising providing each lens in the plurality with essentially the same geometry; and applying to each lens in the plurality a diffractive element having a diffractive power that is different for each lens in the plurality.

A further embodiment is a method of increasing a power of a deformable intraocular lens, comprising applying a diffraction grating to at least one of an anterior surface or a posterior surface of the deformable intraocular lens.

A further embodiment is a plurality of deformable, accommodating intraocular lenses, each lens comprising an anterior surface having an anterior radius; a posterior surface having a posterior radius, disposed opposite the anterior surface and separated from the anterior surface by a thickness, wherein the anterior radii, posterior radii and thicknesses of all lenses in the plurality are essentially equal; and a diffraction element having a diffractive power is disposed on at least one of the anterior or posterior surfaces, wherein the diffractive power is different for each lens in the plurality.

A further embodiment is a plurality of deformable, accommodating intraocular lenses, each lens composing an anterior surface having an anterior radius; a posterior surface having a posterior radius, disposed opposite the anterior surface and separated from the anterior surface by a thickness; a geometry defined by the anterior radius, the posterior radius and the thickness; an accommodation range defined by the geometry, the accommodation ranges of all the lenses in the plurality being essentially equal; and a diffraction grating having a diffractive power, disposed on one of the anterior or posterior surfaces, wherein the diffractive power is different for each lens in the plurality.

A further embodiment is an apparatus for providing a range of nominal powers for a plurality of intraocular lenses, the apparatus comprising means for providing each lens in the plurality with essentially the same geometry; and means for applying to each lens in the plurality a diffractive element having a diffractive power that is different for each lens in the plurality.

These and other aspects of the present application will be apparent from the detailed description below. In no event, however, should the above summaries be construed as limitations on the claimed subject matter, which subject matter is defined solely by the attached claims, as may be amended during prosecution.

BRIEF DESCRIPTION OF THE SEVERAL VIEWS OF THE DRAWINGS

FIG. 1 is a plan drawing of a meniscus optic having a diffraction grating on is posterior surface.

FIG. 2 is a plan drawing of a meniscus optic having a diffraction grating on is anterior surface.

FIG. 3 is a plan drawing of a meniscus optic having a diffraction grating on both its posterior and anterior surfaces.

FIG. 4 is a plan drawing of a plano-convex optic having a diffraction grating on is posterior surface.

FIG. 5 is a plan drawing of a plano-convex optic having a diffraction grating on is anterior surface.

FIG. 6 is a plan drawing of a plano-convex optic having a diffraction grating on both its posterior and anterior surfaces.

FIG. 7 is a plan drawing of a bi-convex optic having a diffraction grating on is posterior surface.

FIG. 8 is a plan drawing of a bi-convex optic having a diffraction grating on is anterior surface.

FIG. 9 is a plan drawing of a bi-convex optic having a diffraction grating on both its posterior and anterior surfaces.

## DETAILED DESCRIPTION OF THE INVENTION

Typically, an eye that surgically receives an intraocular lens is measured to determine the required nominal power of the intraocular lens. Nominal powers typically range from 6 diopters to 33 diopters, with the most common nominal powers between 16 and 25 diopters. However, a full range, such as -10D through +40D, powers may be used. The intraocular lenses are usually provided in a family, with off-the-shelf nominal powers spanning the range of 6 to 33 diopters, commonly in increments of 0.5 diopters. Once a nominal power is determined for a particular eye, a lens is selected from the family of available lenses.

The intraocular lens itself has an optic, which is largely transparent and has a power that varies in response to a deforming force, and a haptic, which holds the optic inside the capsular bag of the eye and transmits a deforming force from the capsular bag to the optic. Each deformable optic has an anterior surface which faces away from the retina, a posterior surface which faces toward the retina, and a center thickness. The anterior surface and the posterior surface each have a radius of curvature. Note that the radii of curvature may be or approach infiniti (i.e. a piano lens surface). The radii of curvature and the center thickness will be collectively referred to hereinafter as the optic geometry. During deformation of the optic, any or all of the radii of curvature and the thickness can vary under the influence of the zonular force, thereby changing the power of the lens, and thereby bringing into focus objects at varying distances from the eye.

The deforming force may be in the form of an expanding or contracting force, largely in the radial direction with respect to the optical axis, and may be applied to the edge or one or more of the faces of the optic.

Alternatively, there may be a significant non-radial component of the deforming force.

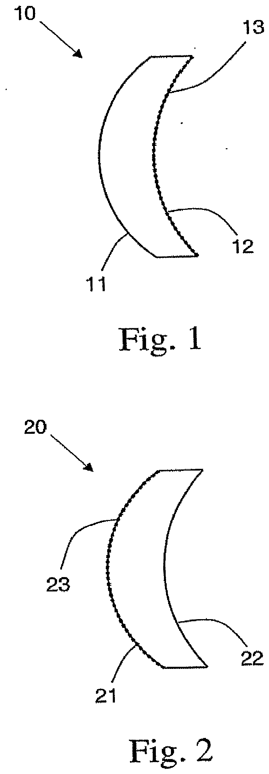

FIG. 1 shows an optic 10 that can provide a sufficient range of accommodation, typically greater than or equal to about 4 diopters, for essentially the entire range of nominal powers. The optic 10 has a meniscus shape, with a convex anterior surface 11 and a concave posterior surface 12. The posterior surface 12 has a diffraction grating 13 that imparts additional optical power to the posterior surface 12. As a result, the optic 10 has both a nominal power contribution from the surface curvatures and from the diffractive grating 13. The power arising from the surface curvatures may be referred to as a refractive power, and the power arising from the grating may be referred to as a diffractive power. In general, as the optic deforms during accommodation, the refractive power varies, while the diffractive power may or may not vary. The grating 13 may be equivalently referred to as a holographic element, a holographic lens, a diffractive lens, or a diffractive element.

The optics for alt the lenses in the family may resemble the optic of FIG. 1, having the same basic shape, but having different amounts of optical power imparted by the diffraction grating 13. The grating 13 may have either positive or negative powers for some or all of the various lenses in the family. Alternatively, a subset of the full family can have the same basic optic shape, with the different nominal powers supplied by different diffractive powers in the grating 13.

The geometry of the optic 10 is defined as the radius of curvature of the anterior surface 11, the radius of curvature of the posterior surface 12, and the axial thickness (or, equivalently, the on-axis thickness) between the anterior and posterior surfaces 11 and 12. A suitable radius of curvature for the anterior surface 11 is 5 mm, although other radii may be used. Likewise, a suitable radius of curvature for the posterior surface 12 is 6 mm, although other radii may be used, such as 7 mm, or any other suitable value. The anterior surface 11 and/or the posterior surface 12 may optionally have one or more aspheric terms and/or a conic term. In addition, the anterior surface and/or the posterior surfaces may have an additional multifocal element, which may be diffractive or refractive in nature, such as a so-called refractive multifocal, or a zonally defined aspheric element.

The geometry of the optic 10 is preferably chosen to maximize the range of accommodation, for a given distorting force. In most cases, this preferred geometry leads to a meniscus-shaped optic 10, which generally has a high efficiency in converting a distorting force into a change in power. This high efficiency may be shown by a geometrical optics-based formula that predicts the total power of the optic, $\Phi$, in terms of the power of the anterior surface, $\Phi_a$, the power of the posterior surface, $\Phi_b$, and the on-axis thickness of the optics, t:

$$\Phi = \Phi_a + \Phi_b - t \cdot \Phi_a \cdot \Phi_b$$

In general, when the optic is distorted to increase its power, it may be squeezed or compressed in a radial manner about its equator, although other methods of distortion may also be used. Under the influence of this compression, the curvatures of the anterior and posterior faces typically become more steep, and the on-axis thickness may optionally increase. For a meniscus-shaped optic 10 in which the anterior face is convex and the posterior face is concave, the first term $\Phi_a$ is positive and increases under compression, the second term $\Phi_b$ is negative and decreases (i.e., becomes more negative) under compression, and the third term (-t: $\Phi_a$ - $\Phi_b$) is positive and increases under compression. The contribution of the third term is significant and beneficial for a meniscus shape; in contrast, it becomes small and relatively unchanging under compression for a plano-convex lens having a flat posterior face; and actually changes in the wrong direction for a bi-convex lens. Hence, for a high efficiency in converting a distorting force to a change in power, the preferred shape for the optic 10 is meniscus. Note that for an optional diffractive element on either or both of the lens surfaces, the radial spacing of the diffraction rings may change under the distorting force of the zonules, potentially leading to an additional change in power. For lens compressing force, the zone width of a diffractive element may decrease, potentially increasing the add power of the lens and contributing to additional changes in the optical performance of the lens.

Note that the power of the optic 10 may also be decreased by an expanding force applied around its equator by the capsular bag. Such an expanding force reduces the curvatures of the anterior and posterior faces 11 and 12, and may optionally decrease the on-axis thickness of the optic 10. Under the influence of such an expanding force, the power of the optic 10 decreases. Additionally, for a diffractive element on either or both of the lens faces, the zone width may increase under the influence of a lens expanding force, potentially decreasing the add power of the lens and contributing to additional changes in the optical performance of the lens.

The meniscus optic 10 has a diffraction grating 13 on its posterior surface 12, which provides an additional amount of nominal power to the optic 10. In this manner, the meniscus optic 10, which is typically used for low nominal powers of approximately 6 to 16 diopters, may be used at much higher nominal powers of up to approximately 33 diopters. The addition of diffractive power to one of the optic surfaces permits the same basic optic geometry to be used for a wide range of nominal powers, with the optic at each nominal power having a full range of accommodation. The optic 10 may be accommodative biased, disaccommodative biased, or in a state between accommodative biased and disaccommodative biased.

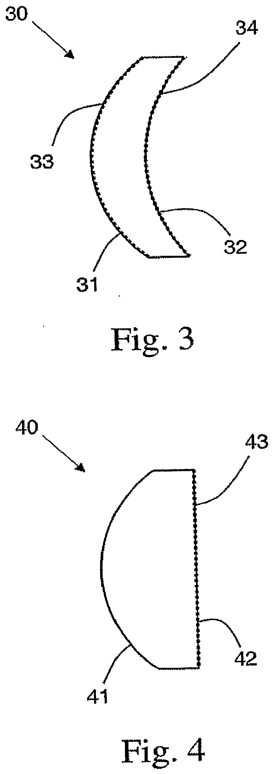

Although the optic 10 shown in FIG. 1 has a diffraction grating 13 on its posterior surface 12, the diffraction grating 23 may also be placed on its anterior surface 21 instead of the posterior surface 22, as in the optic 20 FIG. 2. Alternatively, an optic 30 may have a diffraction grating 33 and 34 on both its anterior surface 31 and its posterior surface 32 as in FIG. 3. Note that although the diffraction gratings are shown covering the entire anterior and posterior faces of the optics, they may extend over only a portion of the faces, such as circular central portions surrounding the optical axis or in one or more annular regions.

For each of the optics in FIGs. 1-3, the geometry is essentially the same for the various lenses in the family, with varying amounts of power supplied by the diffraction gratings. The range of accommodation depends primarily on the geometry of the optic and less so on the diffraction grating, and is sufficiently large for each of the lenses in the family. In contrast, for a common lens family in which the geometry is altered for each nominal power, the range of accommodation may be insufficient at particular nominal powers. In addition, there may be a cost savings in manufacturing the family of lenses, in that a single mold may be used for several optic surfaces in the family.

The diffraction grating may be applied to the optic in a variety of different methods. For instance, the grating may be made integral with the anterior and/or posterior faces of the lens. The grating structure may be first incorporated into a mold, then may be transferred to each face that is fabricated using that particular mold. Or, the grating may be etched or machined into an optic face. Alternatively, the grating may be fabricated external to an optic face, and may be fastened to the optic face after fabrication. For instance, the grating may be fabricated on a sheet or surface that is attached to an optic face. Or, the grating may first be fabricated on a sheet or surface, then the optic is fabricated to lie in contact with the sheet, such as a gel material that is injectable. Preferably, the optic may be made from a silicone or acrylic material, which has a high refractive index, and has a low durometer.

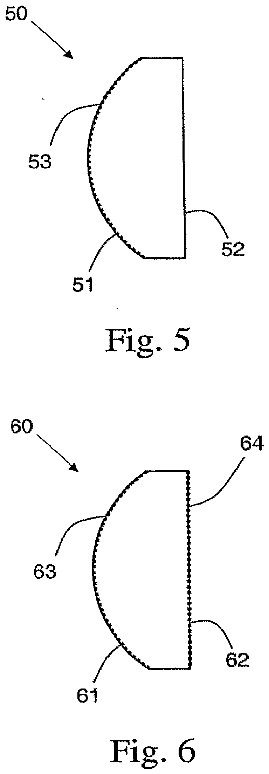

Although meniscus-shaped optics are shown in FIGs. 1-3, a piano-convex or bi-convex optic may be used, so that the amount of power supplied by the diffraction grating may be reduced. A plano-convex optic 40 having a diffraction grating 43 on its flat posterior side 42 but not on its convex anterior side 41 is shown in FIG. 4. A piano-convex optic 50 having a diffraction grating 53 on its convex anterior side 51 but not on its flat posterior side 52 is shown in FIG. 5. FIG. 6 shows a plano-convex optic 60 having a diffraction grating 63 on its convex anterior side 61 and a diffraction grating 64 on its flat posterior side 62. Note that the grating may have either a negative or a positive amount of optical power, as required.

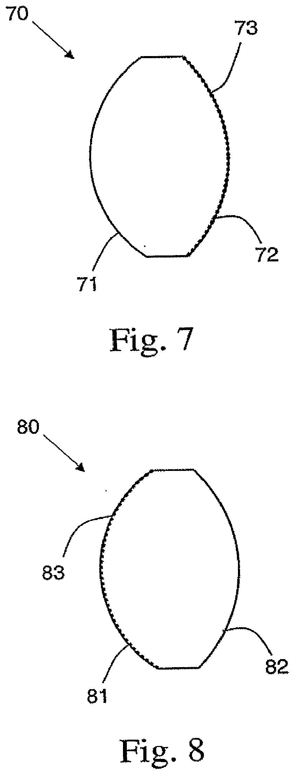

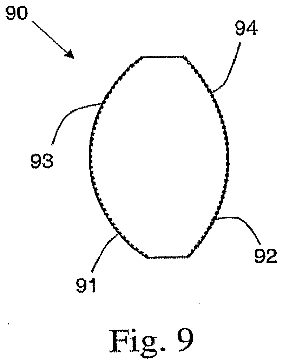

Similarly, bi-convex optics may also be used. FIG. 7 shows a bi-convex optic 70 with a diffraction grating 73 on its posterior face 72 but not its anterior face 71. FIG. 8 shows a bi-convex optic 80 with a diffraction grating 83 on its anterior face 81 but not its posterior face 82. FIG. 9 shows a bi-convex optic 90 with diffraction gratings 93 and 94 on both its anterior face 91 and its posterior face 92. Any or all of the gratings may have either a positive or a negative amount of diffractive power, as required.

The lenses may be designed and specified using a wavelength of 550 nm, which is the wavelength of maximum sensitivity for photopic (light-adapted) vision for the human eye. Alternatively, the lenses may be designed at a wavelength of 510 nm, which is the maximum sensitivity for scotopic (dark-adapted) vision. Likewise, any suitable wavelength in the visible spectrum of about 400 nm to about 700 nm may be used as the design wavelength.

The surface profile of a diffractive element generally has a number of concentric radial rings, with spacings between adjacent rings that decreases at increasing distance away from the center of the lens. The area between adjacent rings is commonly known as a zone. The amount of power added to the lens by the diffractive element is determined in part by the zone diameters. Within each zone, the surface profile has a particular profile height, which may or may not be constant throughout the zone. For instance, the surface profile may increase in height from the inside to the outside of the zone. The surface profile may be defined in order to reduce or counteract the influence of surface deformation on optical performance.

The profile height of the grating affects the order number into which most of the light is diffracted. For instance, if the profile height is roughly one design wavelength, then the diffracted order receiving the most light is the first order. If the profile height is roughly twice the design wavelength, the diffracted second order is most prevalent, and so forth. In general, for a monofocal diffractive element, it is desirable that the profile height should roughly equal an integral multiple of the design wavelength, so that the diffracted light is concentrated primarily in a single diffracted order. In contrast, for a bifocal or multifocal diffractive element, the profile height is unequal to an integral multiple of the design wavelength, so that the light may be divided among two or more diffracted orders.

As an example, a bifocal diffractive lens has a diffractive element in addition to a refractive element. The refractive element brings a distant object into focus, and the diffractive element supplies enough additional power to bring a close object into focus. The diffraction efficiency of the diffraction element is roughly 50%, so that half the light forms a "far" image and half forms a "near" image; both images are always present and are superimposed on the retina. The diffraction efficiency is determined in part by the step height or profile height of the diffractive zones.

In one embodiment, the diffractive surface may have an extended diffractive surface profile, such as the so-called super-zone diffractive lenses disclosed by J. C. Marron, et al, in "Higher-Order Kinoforms", Computer and optically formed holographic optics, I. Cindrich, et al., editor, Proc. SPIE 1211, 62-66 (1990).

The description of the invention and its applications as set forth herein is illustrative and is not intended to limit the scope of the invention. Variations and modifications of the embodiments disclosed herein are possible, and practical alternatives to and equivalents of the various elements of the embodiments would be understood to those of ordinary skill in the art upon study of this patent document. These and other variations and modifications of the embodiments disclosed herein may be made without departing from the scope of the invention.

## Claims

1. A method of providing a range of nominal powers for a plurality of intraocular lenses, the method comprising:

   providing each lens in the plurality with essentially the same geometry; and
   applying to each lens in the plurality a diffractive element (13, 23, 33, 34, 43, 53, 63, 64, 73, 83, 93, 94) having a diffractive power that is different for each lens in the plurality.

2. The method of claim 1, wherein each lens in the plurality has a shape that is meniscus-shaped, plano-convex, or bi-convex.

3. The method of claim 1, wherein the diffractive element (13, 23, 33, 34, 43, 53, 63, 64, 73, 83, 93, 94) is applied to a posterior face (12, 22, 32, 42, 52, 62, 72, 82, 92) of each lens in the plurality or an anterior face (11, 21, 31, 41, 51, 61, 71, 81, 91) of each lens in the plurality.

4. The method of claim 1, wherein each diffractive element (13, 23, 33, 34, 43, 53, 63, 64, 73, 83, 93, 94) has a surface profile height generally equal to the design wavelength.

5. The method of claim 4, wherein each lens has a design wavelength of about 550 nm.

6. A method of increasing a power of a deformable intraocular lens, comprising:

   applying a diffraction grating (13, 23, 33, 34, 44, 53, 63, 64, 73, 83, 93, 94) to at least one of an anterior face (11, 21, 31, 41, 51, 61, 71, 81, 91) or a posterior (12, 22, 32, 42, 52, 62, 72, 82, 92) face of the deformable intraocular lens.

**7.** A plurality of deformable, accommodating intraocular lenses, each lens comprising:

an anterior surface (11, 21, 31, 41, 51, 61, 71, 81, 91) having an anterior radius; a posterior surface (12, 22, 32, 42, 52, 62, 72, 82, 92) having a posterior radius, disposed opposite the anterior surface (11, 21, 31, 41, 51, 61, 71, 81, 91) and separated from the anterior surface by a thickness, wherein the anterior radii, posterior radii and thicknesses of all lenses in the plurality are essentially equal; and a diffraction element (13, 23, 33, 34, 44, 53, 63, 64, 73, 83, 93, 94) having a diffractive power, disposed on at least one of the anterior (11, 21, 31, 41, 51, 61, 71, 81, 91) or posterior (12, 22, 32, 42, 52, 62, 72, 82, 92) surfaces, wherein the diffractive power is different for each lens in the plurality.

**8.** The plurality of deformable, accommodating intraocular lenses of claim 7, wherein the diffraction element (13, 23, 33, 34, 44, 53, 63, 64, 73, 83, 93, 94) comprises at least one diffraction grating (13, 23, 33, 34, 44, 53, 63, 64, 73, 83, 93, 94) applied to at least one of the posterior surface (12, 22, 32, 42, 52, 62, 72, 82, 92) and the anterior surface (11, 21, 31, 41, 51, 61, 71, 81, 91).

**9.** The plurality of deformable, accommodating intraocular lenses of claim 7, wherein the anterior radius, the posterior radius, and the thickness define a refractive power; wherein the refractive power is variable in response to a deforming force; wherein the refractive power varies over a full accommodation range; and wherein the full accommodation range is a power at the lens that brings a distant object into focus, subtracted from a power at the lens that brings a near object into focus.

**10.** The plurality of deformable, accommodating intraocular lenses of claim 9, wherein the full accommodation range is about 4 diopters.

**11.** The plurality of deformable, accommodating intraocular lenses of claim 7, wherein the anterior surface (11, 21, 31, 41, 51, 61, 71, 81, 91) is convex.

**12.** The plurality of deformable, accommodating intraocular lenses of claim 11, wherein the anterior radius is about 5 mm.

**13.** The plurality of deformable, accommodating intraocular lenses of claim 7, wherein the posterior surface (12, 22, 32, 42, 52, 62, 72, 82, 92) is concave.

**14.** The plurality of deformable, accommodating intraocular lenses of claim 7, wherein the posterior surface (12, 22, 32, 42, 52, 62, 72, 82, 92) is flat.

**15.** The plurality of deformable, accommodating intraocular lenses of claim 7, wherein the posterior surface (12, 22, 32, 42, 52, 62, 72, 82, 92) is convex.

**Patentansprüche**

**1.** Verfahren zum Bereitstellen eines Bereichs von Nennstärken für mehrere Intraokularlinsen, wobei das Verfahren Folgendes umfasst:

Bereitstellen jeder Linse der Mehrzahl mit im Wesentlichen derselben Geometrie; und Aufbringen eines beugenden Elements (13, 23, 33, 34, 43, 53, 63, 64, 73, 83, 93, 94), das eine Beugungskraft aufweist, die für jede Linse der Mehrzahl verschieden ist, auf jede Linse der Mehrzahl.

**2.** Verfahren nach Anspruch 1, wobei jede Linse der Mehrzahl eine Form aufweist, die meniskusförmig, plankonvex oder bikonvex ist.

**3.** Verfahren nach Anspruch 1, wobei das beugende Element (13, 23, 33, 34, 43, 53, 63, 64, 73, 83, 93, 94) auf eine posteriore Fläche (12, 22, 32, 42, 52, 62, 72, 82, 92) jeder Linse der Mehrzahl oder auf eine anteriore Fläche (11, 21, 31, 41, 51, 61, 71, 81, 91) jeder Linse der Mehrzahl aufgebracht wird.

**4.** Verfahren nach Anspruch 1, wobei jedes beugende Element (13, 23, 33, 34, 43, 53, 63, 64, 73, 83, 93, 94) eine Oberflächenprofilhöhe aufweist, die im Allgemeinen gleich der Entwurfswellenlänge ist.

**5.** Verfahren nach Anspruch 4, wobei jede Linse eine Entwurfswellenlänge von etwa 550 nm aufweist.

**6.** Verfahren zum Erhöhen einer Stärke einer verformbaren Intraokularlinse, das Folgendes umfasst:

Aufbringen eines Beugungsgitters (13, 23, 33, 34, 44, 53, 63, 64, 73, 83, 93, 94) auf eine anteriore Fläche (11, 21, 31, 41, 51, 61, 71, 81, 91) und/oder auf eine posteriore Fläche (12, 22, 32, 42, 52, 62, 72, 82, 92) der verformbaren Intraokularlinse.

**7.** Mehrere verformbare, akkommodationsfähige Intraokularlinsen, wobei jede Linse Folgendes umfasst:

eine anteriore Oberfläche (11, 21, 31, 41, 51, 61, 71, 81, 91), die einen anterioren Radius aufweist;

eine posteriore Oberfläche (12, 22, 32, 42, 52, 62, 72, 82, 92), die einen posterioren Radius aufweist, die der anterioren Oberfläche (11, 21, 31, 41, 51, 61, 71, 81, 91) gegenüberliegend und durch eine Dicke von der anterioren Oberfläche getrennt angeordnet ist, wobei die anterioren Radien, die posterioren Radien und die Dicken aller Linsen der Mehrzahl im Wesentlichen gleich sind; und

ein Beugungselement (13, 23, 33, 34, 44, 53, 63, 64, 73, 83, 93, 94), das eine Beugungskraft aufweist, das auf der anterioren Oberfläche (11, 21, 31, 41, 51, 61, 71, 81, 91) und/oder auf der posterioren Oberfläche (12, 22, 32, 42, 52, 62, 72, 82, 92) angeordnet ist, wobei die Beugungskraft für jede Linse der Mehrzahl verschieden ist.

8. Mehrere verformbare, akkommodationsfähige Intraokularlinsen nach Anspruch 7, wobei das Beugungselement (13, 23, 33, 34, 44, 53, 63, 64, 73, 83, 93, 94) mindestens ein Beugungsgitter (13, 23, 33, 34, 44, 53, 63, 64, 73, 83, 93, 94) umfasst, das auf der posterioren Oberfläche (12, 22, 32, 42, 52, 62, 72, 82, 92) und/oder auf der anterioren Oberfläche (11, 21, 31, 41, 51, 61, 71, 81, 91) aufgebracht ist.

9. Mehrere verformbare, akkommodationsfähige Intraokularlinsen nach Anspruch 7, wobei der anteriore Radius, der posteriore Radius und die Dicke eine Brechkraft definieren; wobei die Brechkraft als Antwort auf eine verformende Kraft veränderlich ist; wobei sich die Brechkraft über einen vollständigen Akkomodationsbereich ändert; und wobei der vollständige Akkomodationsbereich eine Stärke der Linse, die einen entfernten Gegenstand fokussiert, abgezogen von einer Stärke der Linse, die einen nahen Gegenstand fokussiert, ist.

10. Mehrere verformbare, akkommodationsfähige Intraokularlinsen nach Anspruch 9, wobei der vollständige Akkomodationsbereich etwa 4 Dioptrien beträgt.

11. Mehrere verformbare, akkommodationsfähige Intraokularlinsen nach Anspruch 7, wobei die anteriore Oberfläche (11, 21, 31, 41, 51, 61, 71, 81, 91) konvex ist.

12. Mehrere verformbare, akkommodationsfähige Intraokularlinsen nach Anspruch 11, wobei der anteriore Radius etwa 5 mm beträgt.

13. Mehrere verformbare, akkommodationsfähige Intraokularlinsen nach Anspruch 7, wobei die posteriore Oberfläche (12, 22, 32, 42, 52, 62, 72, 82, 92) konkav ist.

14. Mehrere verformbare, akkommodationsfähige Intraokularlinsen nach Anspruch 7, wobei die posteriore Oberfläche (12, 22, 32, 42, 52, 62, 72, 82, 92) flach ist.

15. Mehrere verformbare, akkommodationsfähige Intraokularlinsen nach Anspruch 7, wobei die posteriore Oberfläche (12, 22, 32, 42, 52, 62, 72, 82, 92) konvex ist.

## Revendications

1. Procédé d'obtention d'une gamme de puissances nominales pour une pluralité de lentilles intraoculaires, le procédé comprenant les étapes suivantes :

   obtenir chaque lentille dans la pluralité avec essentiellement la même géométrie ; et
   appliquer à chaque lentille dans la pluralité un élément diffractif (13, 23, 33, 34, 43, 53, 63, 64, 73, 83, 93, 94) ayant une puissance diffractive qui est différente pour chaque lentille dans la pluralité.

2. Procédé de la revendication 1, dans lequel chaque lentille dans la pluralité a une forme qui est en forme de ménisque, plan-convexe, ou biconvexe.

3. Procédé de la revendication 1, dans lequel l'élément diffractif (13, 23, 33, 34, 43, 53, 63, 64, 73, 83, 93, 94) est appliqué à une face postérieure (12, 22, 32, 42, 52, 62, 72, 82, 92) de chaque lentille dans la pluralité ou une face antérieure (11, 21, 31, 41, 51, 61, 71, 81, 91) de chaque lentille dans la pluralité.

4. Procédé de la revendication 1, dans lequel chaque élément diffractif (13, 23, 33, 34, 43, 53, 63, 64, 73, 83, 93, 94) a une hauteur de profil de surface généralement égale à la longueur d'onde de conception.

5. Procédé de la revendication 4, dans lequel chaque lentille a une longueur d'onde de conception d'environ 550 nm.

6. Procédé d'augmentation d'une puissance d'une lentille intraoculaire déformable, comprenant l'étape suivants :

   appliquer un réseau de diffraction (13, 23, 33, 34, 44, 53, 63, 64, 73, 83, 93, 94) à une face antérieure (11, 21, 31, 41, 51, 61, 71, 81, 91) et/ou une face postérieure (12, 22, 32, 42, 52, 62, 72, 82, 92) de la lentille intraoculaire déformable.

7. Pluralité de lentilles intraoculaires déformables, accommodatives, chaque lentille comprenant :

une surface antérieure (11, 21, 31, 41, 51, 61, 71, 81, 91) ayant un rayon antérieur ;
une surface postérieure (12, 22, 32, 42, 52, 62, 72, 82, 92) ayant un rayon postérieur, disposée à l'opposé de la surface antérieure (11, 21, 31, 41, 51, 61, 71, 81, 91) et séparée de la surface antérieure par une épaisseur, les rayons antérieurs, les rayons postérieurs et les épaisseurs de toutes les lentilles dans la pluralité étant essentiellement égaux ; et
un élément de diffraction (13, 23, 33, 34, 44, 53, 63, 64, 73, 83, 93, 94) ayant une puissance diffractive, disposé sur au moins une des surfaces antérieure (11, 21, 31, 41, 51, 61, 71, 81, 91) ou postérieure (12, 22, 32, 42, 52, 62, 72, 82, 92), la puissance diffractive étant différente pour chaque lentille dans la pluralité.

8. Pluralité de lentilles intraoculaires déformables, accommodatives de la revendication 7, dans laquelle l'élément de diffraction (13, 23, 33, 34, 44, 53, 63, 64, 73, 83, 93, 94) comprend au moins un réseau de diffraction (13, 23, 33, 34, 44, 53, 63, 64, 73, 83, 93, 94) appliqué à la surface postérieure (12, 22, 32, 42, 52, 62, 72, 82, 92) et/ou la surface antérieure (11, 21, 31, 41, 51, 61, 71, 81, 91).

9. Pluralité de lentilles intraoculaires déformables, accommodatives de la revendication 7,
dans laquelle le rayon antérieur, le rayon postérieur et l'épaisseur définissent une puissance réfractive ;
dans laquelle la puissance réfractive est variable en réponse à une force de déformation ;
dans laquelle la puissance réfractive varie sur une gamme d'accommodation complète ; et
dans laquelle la gamme d'accommodation complète est une puissance au niveau de la lentille qui met au point un objet distant, retranchée d'une puissance au niveau de la lentille qui met au point un objet proche.

10. Pluralité de lentilles intraoculaires déformables, accommodatives de la revendication 9, dans laquelle la gamme d'accommodation complète est d'environ 4 dioptries.

11. Pluralité de lentilles intraoculaires déformables, accommodatives de la revendication 7, dans laquelle la surface antérieure (11, 21, 31, 41, 51, 61, 71, 81, 91) est convexe.

12. Pluralité de lentilles intraoculaires déformables, accommodatives de la revendication 11, dans laquelle le rayon antérieur est d'environ 5 mm.

13. Pluralité de lentilles intraoculaires déformables, accommodatives de la revendication 7, dans laquelle la surface postérieure (12, 22, 32, 42, 52, 62, 72, 82, 92) est concave.

14. Pluralité de lentilles intraoculaires déformables, accommodatives de la revendication 7, dans laquelle la surface postérieure (12, 22, 32, 42, 52, 62, 72, 82, 92) est plate.

15. Pluralité de lentilles intraoculaires déformables, accommodatives de la revendication 7, dans laquelle la surface postérieure (12, 22, 32, 42, 52, 62, 72, 82, 92) est convexe.

Fig. 1

Fig. 2

30

34

33

32

31

Fig. 3

40

43

42

41

Fig. 4

50

53

51

52

**Fig. 5**

60

64

63

61

62

**Fig. 6**

70

73

71

72

## Fig. 7

80

83

81

82

## Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005011531 A

**Non-patent literature cited in the description**

- **J. C. MARRON et al.** Higher-Order Kinoforms'', Computer and optically formed holographic optics. *Proc. SPIE,* 1990, vol. 1211, 62-66